# EUROPÄISCHE PATENTSCHRIFT

(11) **EP 3 777 946 B1**
(45) Veröffentlichungstag und Bekanntmachung des Hinweises auf die Patenterteilung: **18.01.2023**
(21) Anmeldenummer: 19191879.6
(22) Anmeldetag: 15.08.2019
(51) Int. Cl.: A61M 25/00, A61B 5/00

(54) **LUER-LOCK-SYSTEM FÜR DIGITALE BALLONKATHETER**
LUER LOCK SYSTEM FOR DIGITAL BALLOON CATHETER
SYSTÈME LUER-LOCK POUR CATHÉTERS À BALLONNET NUMÉRIQUE

(43) Veröffentlichungstag der Anmeldung: 17.02.2021
(73) Patentinhaber: BIOTRONIK AG, 8180 Bülach (CH)
(72) Erfinder: Fargahi, Amir, 8180 Bülach (CH)
(74) Vertreter: Biotronik Corporate Services SE

(56) Entgegenhaltungen:
- US-A1- 2005 059 958
- US-A1- 2007 219 551
- US-A1- 2011 224 649
- US-A1- 2015 105 659
- US-A1- 2016 270 732
- US-A1- 2019 245 310

## Beschreibung

Die Erfindung betrifft einen Katheter, insbesondere einen Ballonkatheter. Die Erfindung wird hauptsächlich am Beispiel eines Ballonkatheters zur Durchführung einer Angioplastie oder Implantation eines Stents beschrieben, ist jedoch nicht darauf beschränkt sondern für jeden vaskulären Katheter geeignet.

Ballonkatheter werden z.B. eingesetzt, um im Rahmen einer Angioplastie eine Stenose mittels eines Stents aufzuweiten, der auf einem Ballon des Ballonkatheters angeordnet ist und mittels des Ballonkatheters zum Zielort transportiert wird und dort mittels des Ballons aufgeweitet wird bzw. im Gefäß zur Beseitigung der Engstelle verankert wird. Die vorliegende Erfindung ist jedoch auch auf andere Katheter anwendbar.

Bei derartigen Kathetern besteht oftmals die Herausforderung, eine auf dem Katheter bzw. Ballon angeordnete elektronische Komponente, z.B. einen Sensor zum Messen eines Durchmessers des Ballons, geeignet elektrisch zu kontaktieren.

Oftmals werden hierbei in komplexen Verfahren und Lötvorgängen elektrische Leitungen entlang des Katheters verlegt und über Lötverbindungen mit elektrischen Anschlüssen bzw. Steckern am proximalen Ende des Katheters verbunden.

Hierbei besteht insbesondere bei gedruckten elektrischen Leitern die Problematik, dass sich derartige elektrische Leiter aus gedruckten Tinten nur äußerst schwierig über herkömmliche Lötverfahren kontaktieren lassen. Lötverfahren sind dabei insbesondere aufgrund der hohen Temperaturen wegen der vergleichsweise temperatursensitiven Kunststoffmaterialien von Kathetern problematisch. Siehe zum Beispiel US2015105659, US2016270732, US2007219551 oder US2019245310.

Der vorliegenden Erfindung liegt daher hiervon ausgehend die Aufgabe zugrunde, einen Katheter und ein Verfahren zur Herstellung eines solchen Katheters bereitzustellen, der bzw. das hinsichtlich der oben dargelegten Problematik verbessert ist.

Diese Aufgabe wird durch einen Katheter mit den Merkmalen des Anspruchs 1 gelöst. Vorteilhafte Ausgestaltungen des Katheters sind in den entsprechenden Unteransprüchen angegeben und werden nachfolgend beschrieben.

Gemäß Anspruch 1 wird ein Katheter offenbart, mit:
- einem Katheterschaft, der sich von einem proximalen Endabschnitt zu einem distalen Endabschnitt des Katheterschafts erstreckt und eine Außenseite aufweist, und
- einem mit dem proximalen Endabschnitt des Katheterschaftes verbundenen Verbinder.

Erfindungsgemäß ist hierbei vorgesehen, dass der Verbinder ein Material aufweist, mit dem der proximale Endabschnitt des Katheterschaftes umgeben ist, wobei auf die Außenseite des Katheterschaftes zumindest ein elektrischer Leiter aufgedruckt ist, wobei der mindestens eine elektrische Leiter eine Leiterbahn einer Leiterplatte mechanisch kontaktiert, die in das Material des Verbinders eingebettet ist, so dass der mindestens eine elektrische Leiter und die mindestens eine Leiterbahn elektrisch leitend verbunden sind.

Gemäß einer Ausführungsform ist der Erfindung steht der Verbinder mit einem Lumen des Katheterschaftes in Strömungsverbindung, so dass über den Verbinder ein fluides Medium in das Lumen des Katherschaftes einleitbar ist. Dies gilt insbesondere, wenn der Katheterschaft als Außenschaft eines Ballonkatheters ausgebildet ist. In dem Fall wird über den Verbinder ein Fluid zum Befüllen des Ballons in das Lumen des Außenschaft geführt. Alternativ kann der Verbinder am proximalen Ende eines Innenschaftes angeordnet sein und derart ausgestaltet sein, dass ein fluides Medium zum Spülen des Lumens des Innenschaftes einleitbar ist.

Bei einem Katheter zur Implantation einer selbstexpandierenden Prothese (z.B. ein selbstexpandierender Stent) kann der Verbinder am proximalen Ende eines Außenschaftes und/oder eines Innenschaftes angeordnet werden. Auch in diesen Fällen kann der Verbinder mit dem Lumen des Außen- und/oder Innenschaftes derart in Strömungsverbindung stehen, dass über den Verbinder ein fluides Medium zum Spülen der Lumen einleitbar ist.

Besonders bevorzugt ist vorgesehen, dass es sich bei dem Verbinder um einen Luer-Lock-Verbinder handelt.

Mit dem erfindungsgemäßen Design wird bei den genannten Ausgestaltungen somit auf vorteilhafte Weise die aktuelle Inflation-Deflation-Funktion oder Spül-Funktion der Verbinder, insbesondere der Luer-Lock-Verbindungen, mit einem integrierten (mit der Leiterplatte verbundenen) Steckverbinder und ggf. einem dazu gehörenden Konnektor ergänzt.

Diese Erfindung beschreibt somit ein hochintegriertes Konzept zur sicheren und preiswerten elektrischen Verbindungen zwischen Leiterbahnen und gedruckten elektrisch leitenden Tinten.

Gemäß einer bevorzugten Ausführungsform der Erfindung ist vorgesehen, dass der proximale Endabschnitt des Katheterschaftes mit dem Material des Verbinders umspritzt ist.

Weiterhin ist gemäß einer bevorzugten Ausführungsform der Erfindung vorgesehen, dass das Material des Verbinders ein thermoplastische Kunststoff ist.

Im Prinzip kommen für die Erfindung alle thermoplastischen Kunststoffe in Frage, die für Medizinprodukte geeignet sind und in einem Spritzgußverfahren verarbeitet werden können, dessen Temperatur die Funktionalität der Leiterbahnen oder einer Leiterplatte nicht beeinflußt. Dies sind insbesondere ABS (Acrylnitrile butaden styrene), ABS-PC (Acrylnitrile butaden styrene + polycarbonate blend), ANMA (Acrlynitirle methyl acrylate copolymer), CAP (Cellulose acetetate propionate), COC (cyclic olefin copolymer), Copolyester, LCP (Liquid crystal polyester), MABS (Methylmethacrylate acrylnitirle butadien styrene), Polyamide (transparent, PA11, PA12, PA6, PA66), PBT (Polybutylene terephthalate), PC (Polycarbonate), PC + Polyester (Polycarbonate + polyester blend), PCTA (Polycyclohexylenedimethylene tere/isophthalate copolyester), PCTG (Polycyclohexylenedimethylene terephthalate glycol copolymer), HDPE (high density polyethylene), LLDPE (linear low density polyethylene), MDPE (medium denisty Polyethylene), PEBA (Polyester blockamide, thermoplastic polyamide-polyether elastomer mit verschiedener Shore Härte), PEI (Polyether imide), PEI+ PCE (Polyetherimide + polycarbonate ester blend), PET (Polyethylene terephthalate), PFA (Perfluorakoxyethylene), PGA (Polyglycolic acid), PLA (Polylactic acid), PMMA (Polymethylmethacylate), PMP (Polymethylpentene), POM (Polyoxymethylene), PP (Polypropylene), PPE + PS (Polyphenylene ether + Polystyrene), PPSU (Polyphenylsulfione), PS (Polystyrene), PSU (Polysulfone), PVC (Polyvinly chloride, pasticized), PCC-P (Polyvinyl chloride elastomer, pasticized), PVDF (Polyvinylidene difluoride), SAN (Acrylnitrile styrene coploymer), SB (Styrene butadiene copolymer), SEBS (Styrene ethylene butylenen styrene block copolymer, verschiedener Härte), SMMA (Styrene methyl methycrylate copolymer), SRP (self reinforcing polyphenylene), TPC (Thermoplastic copolyester-ether elastomer verschiedener Härte)), TPU (thermoplastic polyurethane elastomer verschiedener Härte), TPV (thermoplastic vulcanizate, verschiedener Härte).

Weiterhin ist gemäß einer bevorzugten Ausführungsform der Erfindung vorgesehen, dass der mindestens eine elektrische Leiter und die Leiterbahn überlappend angeordnet sind, wobei vorzugsweise die Leiterbahn und der mindestens eine elektrische Leiter auf der Außenseite des Katheterschaftes in einer gemeinsamen Längserstreckungsrichtung einander überlappen, also insbesondere nicht einander kreuzend angeordnet sind.

Weiterhin ist gemäß einer bevorzugten Ausführungsform der Erfindung vorgesehen, dass die Leiterbahn in einem Bereich der Leiterbahn, in dem die Leiterbahn mit dem mindestens einen elektrischen Leiter überlappt, auf einer Oberfläche der Leiterplatte angeordnet ist, wobei die Oberfläche der Außenseite des Katheterschaftes zugewandt ist bzw. auf dieser angeordnet ist.

Abseits der Überlappung bzw. des Kontaktes zwischen dem mindestens einen elektrischen Leiter und der Leiterbahn sind der mindestens eine elektrische Leiter und die mindestens eine Leiterbahn vorzugsweise geeignet elektrisch isoliert. Bei der Leiterplatte kann diese Isolierung durch das umgebende Material des Verbinders sichergestellt werden.

Weiterhin ist gemäß einer bevorzugten Ausführungsform der Erfindung vorgesehen, dass die Leiterplatte einen ersten und einen damit verbundenen zweiten Abschnitt aufweist, wobei die beiden Abschnitte winklig zueinander angeordnet sind, insbesondere rechtwinklig. Diese Ausgestaltung erlaubt eine besonders einfache Kontaktierung bzw. Verbindung des Katheters, insbesondere über einen Steckverbinder, mit einer Einrichtung zur Applikation, Auswertung und/oder Steuerung elektrischer Signale.

Weiterhin ist gemäß einer bevorzugten Ausführungsform der Erfindung vorgesehen, dass der besagte Bereich der Leiterbahn an einem Ende des ersten Abschnitts angeordnet ist.

Weiterhin ist gemäß einer bevorzugten Ausführungsform der Erfindung vorgesehen, dass die Leiterplatte mit einem Steckverbinder verbunden ist, über den die Leiterbahn elektrisch leitend kontaktierbar ist. Bevorzugt ist hierbei der Steckverbinder mit einem Endabschnitt des zweiten Abschnitts der Leiterplatte verbunden, insbesondere durch eine Lötverbindung. Aufgrund der winkligen Anordnung bzw. Ausgestaltung der beiden Abschnitte der Leiterplatte kann der Steckverbinder insbesondere quer bzw. in einem Winkel zur axialen Richtung des Katheters aus dem Verbinder herausgeführt sein.

Weiterhin ist gemäß einer bevorzugten Ausführungsform der Erfindung vorgesehen, dass die Leiterbahn in dem besagte Bereich der Leiterbahn, der mit dem mindestens einen elektrischen Leiter überlappt, eine Breite aufweist, die größer ist als eine Breite des mindestens einen elektrischen Leiters. Hier ist jeweils die Breite senkrecht zur Längserstreckungsrichtung der Leiterbahn bzw. des Leiters gemeint.

Weiterhin ist gemäß einer bevorzugten Ausführungsform der Erfindung vorgesehen, dass die Leiterplatte aus Polyimid gebildet ist. Weiterhin kann die Leiterplatte eine Stärke im Bereich von z. B. 0,03 mm bis 0,1 mm aufweisen.

Vorzugsweise handelt es sich bei dem Katheter um einen Ballonkatheter, wobei insbesondere der mindestens eine elektrische Leiter mit einem Sensor des Ballonkatheters verbunden ist, insbesondere mit einem auf einem Ballon des Ballonkatheters angeordneten Sensor.

Vorstehend wurde die Erfindung anhand einer Leiterbahn und eines gedruckten elektrischen Leiters des Katheterschaftes beschrieben. Es können natürlich eine Mehrzahl an separaten Leiterbahnen der Leiterplatte und eine Mehrzahl an gedruckten Leitern des Katheterschaftes in der hierein beschriebenen Weise miteinander mechanisch und damit elektrisch leitend in Kontakt stehen.

Ein weiterer Aspekt der vorliegenden Erfindung betrifft ein Verfahren zur Herstellung eines Katheters (insbesondere zur Herstellung eines erfindungsgemäßen Katheters), aufweisend die Schritte:
- Aufdrucken zumindest eines elektrischen Leiters auf eine Außenseite eines Katheterschaftes des Katheters mit einer elektrisch leitfähigen Tinte,
- Bereitstellen einer Leiterplatte, die zumindest eine Leiterbahn aufweist,
- Anordnen der Leiterplatte auf der Außenseite des Katheterschaftes im Bereich eines proximalen Endabschnitts des Außenschaftes, so dass die Leiterbahn den mindestens einen elektrischen Leiter mechanisch und damit elektrisch leitend kontaktiert und insbesondere mit diesem überlappt, und
- Umgeben der Leiterplatte und des proximalen Endabschnitts des Katheterschaftes mit einem Material zum Ausbilden eines mit dem proximalen Endabschnitt des Außenschaftes verbundenen Verbinders.

Hinsichtlich des Anordnens der Leiterplatte bezüglich der Außenseite des Katheterschaftes kann weiterhin vorgesehen sein, dass die Leiterplatte auf der Außenseite fixiert wird, z.B. mittels eines Schrumpfschlauchs.

Gemäß einer Ausführungsform des Verfahrens ist vorzugsweise vorgesehen, dass ein Steckverbinder mit der Leiterplatte verbunden wird, so dass die Leiterbahn (und damit später der mindestens eine gedruckte elektrische Leiter) über den Steckverbinder elektrisch kontaktierbar ist, wobei insbesondere der Steckverbinder mit der Leiterplatte verbunden wird, bevor die Leiterplatte auf der Außenseite des Katheterschaftes angeordnet wird (sowie insbesondere bevor die Leiterplatte und der proximale Endabschnitt des Katheterschaftes mit dem Material des Verbinders umgeben werden).

Gemäß einer weiteren Ausführungsform des Verfahrens ist vorgesehen, dass zum Umgeben des proximalen Endabschnitts des Katheterschaftes und der Leiterplatte mit dem Material des Verbinders der proximale Endabschnitt des Katheterschaftes und die Leiterplatte in einer Form (z. B. Spritzgussform) angeordnet werden und in der Form mit dem Material umgeben werden, insbesondere mittels Spritzgießen umspritzt werden, d. h., in das Material durch Spritzgießen eingegossen werden.

Auch bei dem Verfahren können natürlich mehrere Leiterbahnen der Leiterplatte mit je einem elektrischen Leiter des Katheterschaftes verbunden bzw. kontaktiert werden.

Im Folgenden sollen Ausführungsformen der Erfindung sowie weitere Merkmale und Vorteile der Erfindung anhand der Figuren erläutert werden. Es zeigen:
- Fig. 1: eine schematische Ausführungsform eines erfindungsgemäßen Katheters,
- Fig. 2: eine perspektivische Ansicht eines Außenschaftes des in der Fig. 1 gezeigten Katheters, wobei auf eine Außenseite des Außenschaftes elektrische Leiter aus einer elektrisch leitfähigen Tinte aufgedruckt sind,
- Fig. 3: eine Draufsicht auf eine Oberfläche einer Leiterplatte des in den Figuren 1 und 2 gezeigten Katheters, wobei auf der Oberfläche Leiterbahnen angeordnet sind,
- Fig. 4: eine schematische Ansicht des Anordnens der Leiterplatte auf der Außenseite des Außenschaftes, so dass die Leiterbahnen auf der Oberfläche der Leiterplatte die Leiter auf der Außenseite des Außenschaftes kontaktieren und mit diesen überlappen (vgl. Detail der Figur 4),
- Fig. 5: eine schematische Ansicht des fertigen Katheters, wobei ein Verbinder, insbesondere in Form eines Luer-Lock-Verbinders auf die Anordnung gemäß Figur 4 mittels Spritzgießen aufgebracht worden ist, so dass der Verbinder die Leiterplatte und den proximalen Endabschnitt des Außenschaftes umgibt und die Leiterplatte bezüglich des Außenschaftes festlegt, und zwar unter Sicherstellung der elektrischen Kontakte zwischen den Leiterbahnen und dem jeweils zugeordneten gedruckten Leiter des Außenschaftes,
- Fig. 6: eine schematische Schnittansicht des Verbinders entlang der Linie A-A der Figur 5, und
- Fig. 7: eine weitere schematische Ausführungsform eines erfindungsgemäßen Katheters zur Implantation eines selbstexpandierenden Stents.

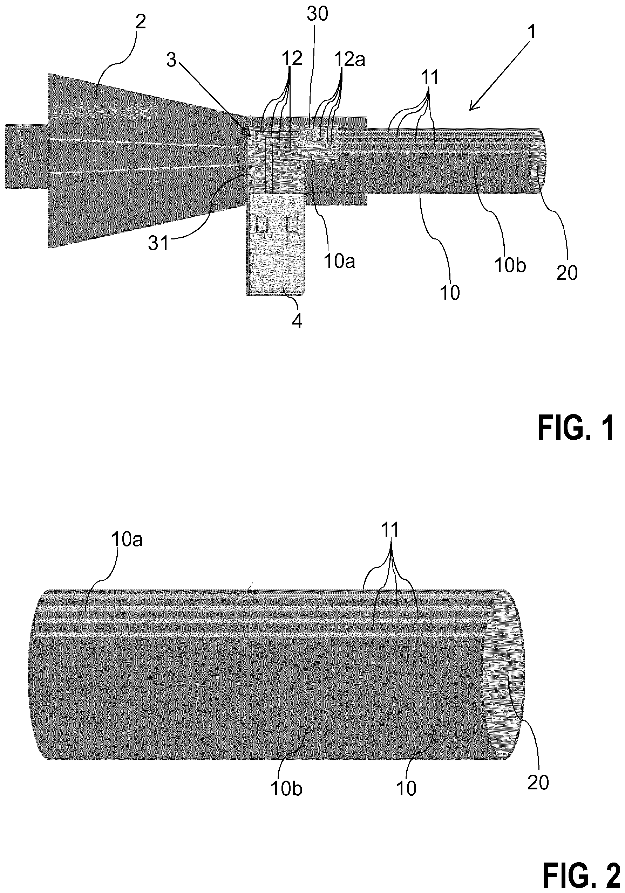

Figur 1 zeigt im Zusammenhang mit Figur 2 eine Ausführungsform eines erfindungsgemäßen Katheters 1, mit einem Außenschaft 10, der sich von einem proximalen Endabschnitt 10a zu einem distalen Endabschnitt (abgeschnitten, nicht dargestellt) des Außenschafts 10 erstreckt und eine Außenseite 10b aufweist. Proximal und distal bedeuten hierbei in der üblichen Weise, dass der proximale Endabschnitt 10a näher am Arzt bzw. Verwender des Katheters 1 gelegen ist, wobei der Außenschaft 10 mit dem distalen Endabschnitt voran in das betreffende Gefäß des Patienten eingeführt wird.

Der Katheter 1 weist weiterhin einen Verbinder 2 auf, der mit dem proximalen Endabschnitt 10a des Außenschaftes 10 verbunden ist bzw. an diesen angespritzt oder geeignet angeformt ist und zur Herstellung einer fluidleitenden Strömungsverbindung mit einem korrespondierenden weiteren Verbinder ausgebildet ist.

Der Verbinder 2 kann z.B. als Luer-Lock-Verbinder ausgebildet sein und ist mit einem Lumen 20 des Außenschafts 10 fluidtechnisch verbunden, durch das z.B. ein Strömungsmittel in einen distal mit dem Außenschaft 10 verbundenen Ballon einleitbar ist (z.B. zum Entfalten des Ballons).

Vorzugsweise weist der Verbinder 2 ein z.B. durch Spritzgießen oder eine vergleichbares Formgebungsverfahren verarbeitetes Material auf bzw. ist aus diesem gebildet, in das der proximale Endabschnitt 10a des Außenschaftes 10 zusammen mit einer Leiterplatte 3 eingebettet ist, wobei auf die Außenseite 10b des Außenschaftes 10 elektrische Leiter 11 aufgedruckt sind, die jeweils eine zugeordnete Leiterbahn 12 der Leiterplatte 3 kontaktieren, so dass die Leiter 11 jeweils elektrisch leitend mit der jeweils zugeordneten Leiterbahn 12 verbunden sind. Bei den Leitern 11 handelt es sich um aus einer elektrisch leitfähigen Tinte gedruckte Leiter 11.

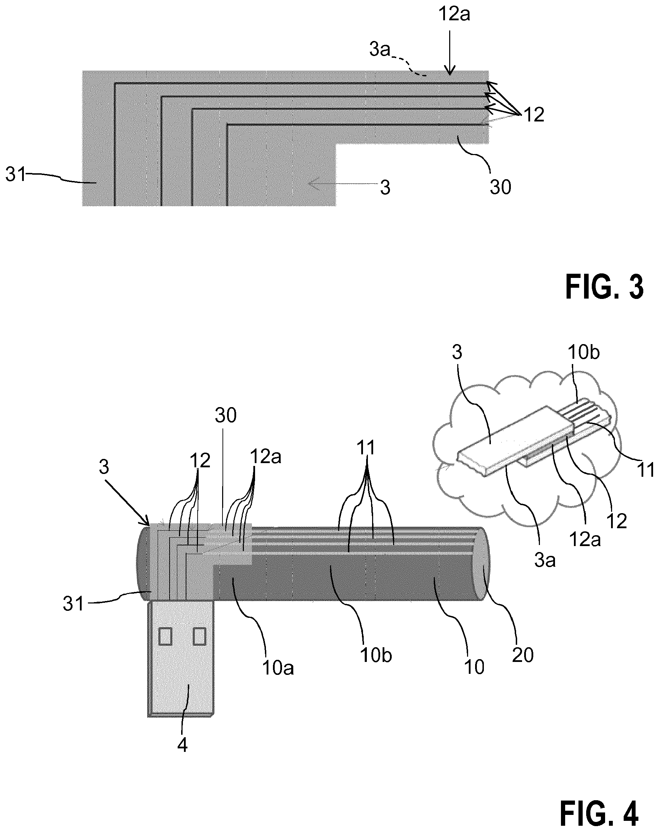

Wie insbesondere anhand des Details der Figur 4 ersichtlich ist, ist vorgesehen, dass die elektrischen Leiter 11 und die Leiterbahnen 12 überlappend angeordnet sind, und zwar vorzugsweise jeweils in der gemeinsamen Längserstreckungsrichtung.

Mit anderen Worten weisen die Leiterbahnen 12 jeweils einem Bereich 12a auf, in dem die jeweilige Leiterbahn 12 an einer Oberfläche 3a der Leiterplatte 3 angeordnet ist und dort mit dem zugeordneten gedruckten Leiter 11 überlappt, so dass elektrisch leitfähige Kontakte zwischen den Leiterbahnen 12 und den Leitern 11 resultieren. Dabei ist die Oberfläche 3a der Leiterplatte 3 der Außenseite 10b des Außenschaftes 10, auf der die Leiter 11 gedruckt sind, zugewandt. In den Figuren 1 und 4 würde die Leiterplatte 3 eigentlich die darunterliegenden Abschnitte der Leiter 11 bzw. Leiterbahnen 12 verdecken. Diese sind dennoch zur Kenntlichmachung der Struktur eingezeichnet.

Die Leiterplatte 3 weist vorzugsweise eine winklige Gestalt auf, wobei ein erster Abschnitt 30 der Leiterplatte 3 winklig, insbesondere rechtwinklig, zu einem zweiten Abschnitt 31 der Leiterplatte 3 verläuft.

Die besagten Überlappungsbereiche 12a sind dabei an einem Ende des ersten Abschnitts 30 vorgesehen, wohingegen ein Endabschnitt des zweiten Abschnitts 31 der Leiterplatte 3 mit einem Steckverbinder 4 verbunden ist, über den die Leiterbahnen 12 (und damit die Leiter 11) elektrisch leitend kontaktierbar sind. Vorzugsweise weisen die Leiterbahnen 12 zumindest in den Bereichen 12a eine Breite auf, die größer ist als eine Breite der Leiter 11 senkrecht zur Längserstreckungsrichtung der Leiter 11 bzw. Leiterbahnen 12.

Die Leiterplatte 3 kann z.B. aus einem Polyimid gebildet sein und kann eine Stärke im Bereich von 0,03 mm bis 0,1 mm aufweisen, insbesondere 0,05 mm. Die Leiterbahnen 12 können in bekannter Weise geätzt sein.

Vorzugsweise wird der männliche oder weibliche Steckverbinder 4 vorab mit einer Leiterplatte 3 vorbereitet. Durch den Spritzprozess (oder durch ein vergleichbares Formgebungsverfahrens zur Erzeugung des Verbinders 2) werden die Kontakt-Verbindungen zwischen den Leiterbahnen 12 der Leiterplatte 3 und den gedruckten Tinten-Leitungen 11 aufgrund des Drucks und des Formschlusses des Materials des Verbinders 2 realisiert bzw. aufrecht erhalten.

Ein möglicher Ablauf des Verfahrens zur Herstellung des Katheters 1 ist in den Figuren 2 bis 6 wiedergegeben.

Zunächst kann der Außenschaft 10 des Katheters 1 vorbereitet werden, indem die nötigen Leiter 11 mit Hilfe von elektrisch leitender Tinte auf die Außenseite 10b des Außenschafts gedruckt werden. Die Breite, Dicke und das Tintenmaterial sind dabei je nach Anforderungen variierbar (vgl. Figur 2).

Sodann wird die Leiterplatte 3 bereitgestellt (z.B. aus Polyimid-Folie, 0,05 mm dick) und die nötigen Leiterbahnen 12 werden geätzt bzw. sind darauf ausgebildet. Die Breite der Leiterbahnen 12 soll dabei vorzugsweise größer als die Breite der gedruckten Leitungen 11 sein (vgl. Fig. 3).

Weiterhin wird die Leiterplatte 3 mit einem Steckverbinder 4 verbunden (z.B. durch Löten). Als Steckverbinder 4 kann z.B. ein HDMI-D-Stecker mit 6,4 mm Breite und 2,8 mm Höhe verwendet werden.

Die Leiterplatte 3 wird dann zusammen mit dem Steckverbinder 4 gemäß Figur 4 auf der Außenseite 10b des Außenschaftes 10 fixiert (z.B. mittels eines Schrumpfschlauches), so dass die oben beschriebene Überlappung zwischen den Leiterbahnen 12 und den gedruckten Leitungen 11 auf dem Außenschaft 10 resultiert.

Die vorbereitete Anordnung gemäß Figur 4 (Leiterplatte 3 mit Steckverbinder 4 und Außenschaft 10) wird schließlich in einer Spritzgussform angeordnet und der Verbinder 2 wird an die Anordnung angespritzt. Der vorzugsweise aus dünnen Polytetrafluorethylen ausgeführte Schrumpfschlauch wird dabei nicht entfernt sondern mit umspritzt.

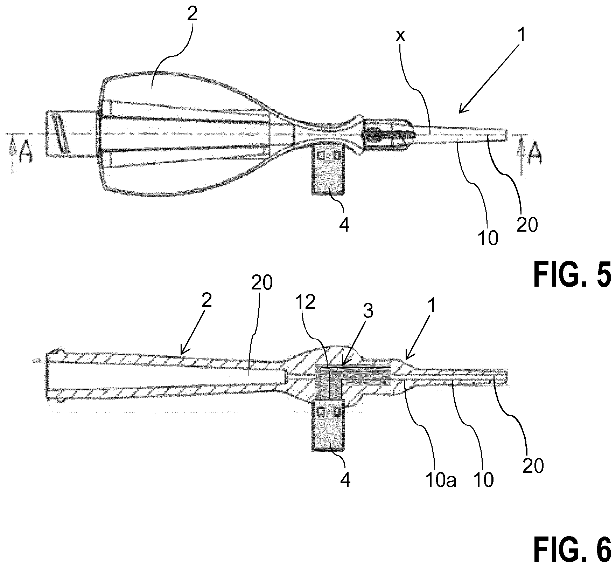

Eine mögliche Ausgestaltung des Verbinders 2 ist in den Figuren 5 und 6 dargestellt, wobei hier der Verbinder 2 als Luer-Lock-Verbinder ausgestaltet ist. Andere Verbindertypen sind natürlich auch mit der Erfindung realisierbar.

Die Figuren 5 und 6 zeigen jeweils den Steckverbinder 4 der besseren Sichtbarkeit halber schematisch in der Blattebene erstreckt. In der Figur 6 würde sich der Steckverbinder 4 eigentlich senkrecht zur Blattebene erstrecken.

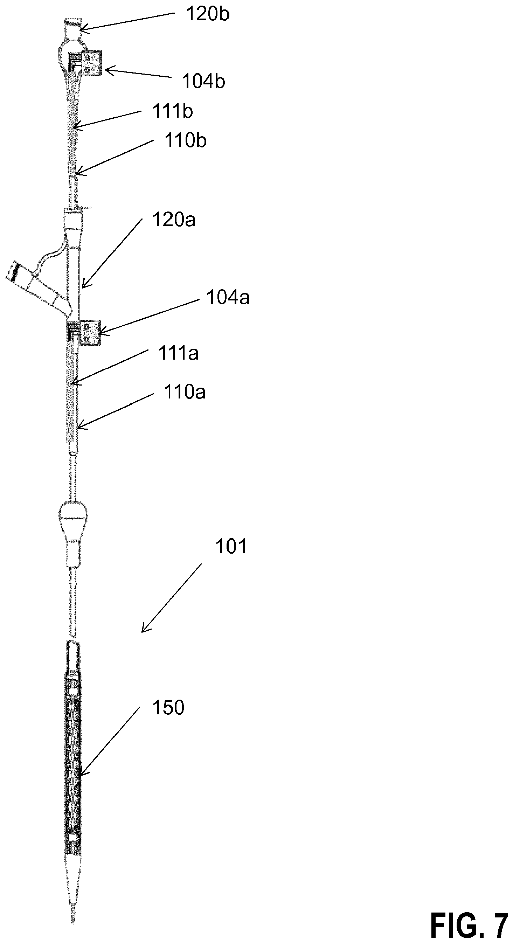

Figur 7 zeigt eine Ausführungsform der Erfindung als Katheter 101 zur Implantation eines selbstexpandierbaren Stents 150. Der Katheter 101 weist zwei gegeneinander bewegliche Katheterschäfte 110a und 110b auf, wobei durch Zurückziehen des Außenschaftes 110a der zwischen Außen- und Innenschaft 110b angeordnete selbstexpandierende Stent 150 freigesetzt wird.

In dieser Ausführungsform der Erfindung weisen sowohl der Außenschaft 110a als auch der Innenschaft 110b an ihrem proximalen Ende einen Verbinder 120a bzw. 120b auf. Die beiden Verbinder 120a, 120b sind derart ausgestaltet, dass über sie ein Fluid zum Spülen des Lumens eingeleitet werden kann (Verbinder 120a ist zur Zufuhr eines Fluids zum Spülen des Lumens zwischen Außenschaft 110a und Innenschaft 110b ausgestaltet, Verbinder 120b ist zur Zufuhr eines Fluids zum Spülen des Lumens des Innenschaftes 110b ausgestaltet).

Die Verbinder 120a und 120b sind analog zur Ausführungsform nach den Figuren 1 bis 4 zur Verbindung der aufgedruckten elektrischen Leiter 111a und 111b auf den Katheterschäften 110a und 10b über eine Leiterplatte mit den jeweiligen Steckverbindern 104a und 104b ausgestaltet.

Die vorliegende Erfindung bietet mit Vorteil ein "two in one Design", bei dem z.B. ein Luer-Anschluss mit einer zweiten Funktion (elektrische Verbindung zwischen Leiterplatte/Steckverbinder und gedruckten Leitungen) bereitgestellt wird, was das Handling für den Arzt verbessert.

Die Erfindung ermöglicht weiterhin eine sichere elektrische Verbindung zwischen katheterseitigen gedruckten Leitungen und einer damit zu verbindenden externen Komponente (z.B. Messgerät, Stromversorgung etc.).

## Patentansprüche

1. Katheter (1), mit:
- einem Katheterschaft (10, 110a, 110b), der sich von einem proximalen Endabschnitt (10a) zu einem distalen Endabschnitt des Katheterschafts (10, 110a, 110b) erstreckt und eine Außenseite (10b) aufweist, und
- einem mit dem proximalen Endabschnitt (10a) des Katheterschaftes (10, 110a, 110b) verbundenen Verbinder (2, 120a, 120b), ,
**dadurch gekennzeichnet, dass**
der Verbinder (2, 120a, 120b) ein Material aufweist, mit dem der proximale Endabschnitt (10a) des Katheterschaftes (10, 110a, 110b) umgeben ist, wobei auf die Außenseite (10b) des Katheterschaftes (10, 110a, 110b) zumindest ein elektrischer Leiter (11, 111a, 111b) aufgedruckt ist, wobei der mindestens eine elektrische Leiter (11, 111a, 111b) eine Leiterbahn (12) einer Leiterplatte (3) kontaktiert, die in das Material des Verbinders (2, 120a, 120b) eingebettet ist, so dass der mindestens eine elektrische Leiter (11, 111a, 111b) und die mindestens eine Leiterbahn (12) elektrisch leitend verbunden sind.

2. Katheter nach Anspruch 1, **dadurch gekennzeichnet, dass** der mindestens eine elektrische Leiter (11, 111a, 111b) aus einer ausgehärteten elektrisch leitfähigen Tinte gebildet ist.

3. Katheter nach Anspruch 1 oder 2, **dadurch gekennzeichnet, dass** der proximale Endabschnitt (10a) des Katheterschaftes (10, 110a, 110b) mit dem Material des Verbinders (2, 120a, 120b) umspritzt ist.

4. Katheter nach einem der vorhergehenden Ansprüche, **dadurch gekennzeichnet, dass** das Material des Verbinders (2, 120a, 120b) aus einem thermoplastischen Kunststoff ist.

5. Katheter nach einem der vorhergehenden Ansprüche, **dadurch gekennzeichnet, dass** der mindestens eine elektrische Leiter (11, 111a, 111b) und die Leiterbahn (12) überlappend angeordnet sind.

6. Katheter nach Anspruch 5, **dadurch gekennzeichnet, dass** die Leiterbahn (12) in einem Bereich (12a) der Leiterbahn (12), in dem die Leiterbahn (12) mit dem mindestens einen elektrischen Leiter (11, 111a, 111b) überlappt, auf einer Oberfläche (3a) der Leiterplatte (3) angeordnet ist, wobei die Oberfläche (3a) der Außenseite (10b) des Katheterschaftes (10, 110a, 110b) zugewandt ist.

7. Katheter nach einem der vorhergehenden Ansprüche, **dadurch gekennzeichnet, dass** die Leiterplatte (3) einen ersten und einen damit verbundenen zweiten Abschnitt (30, 31) aufweist, wobei die beiden Abschnitte (30, 31) winklig zueinander angeordnet sind.

8. Katheter nach Anspruch 6 oder nach Anspruch 7 sofern rückbezogen auf Anspruch 6, **dadurch gekennzeichnet, dass** der besagte Bereich (12a) der Leiterbahn (12) an einem Ende des ersten Abschnitts (30) angeordnet ist.

9. Katheter nach einem der vorhergehenden Ansprüche, **dadurch gekennzeichnet, dass** die Leiterplatte (3) mit einem Steckverbinder (4, 104a, 104b) verbunden ist, über den die Leiterbahn (12) elektrisch leitend kontaktierbar ist.

10. Katheter nach den Ansprüchen 7 und 9, **dadurch gekennzeichnet dass** der Steckverbinder (4, 104a, 104b) mit einem Endabschnitt des zweiten Abschnitts (31) der Leiterplatte (3) verbunden ist.

11. Katheter nach Anspruch 6 oder nach einem der Ansprüche 7 bis 10 soweit rückbezogen auf Anspruch 6, **dadurch gekennzeichnet, dass** die Leiterbahn (12) in dem besagte Bereich (12a) der Leiterbahn (12) eine Breite aufweist, die größer ist als eine Breite des mindestens einen elektrischen Leiters (11, 111a, 111b).

12. Katheter nach einem der vorhergehenden Ansprüche, **dadurch gekennzeichnet dass** die Leiterplatte (3) aus Polyimid gebildet ist und/oder dass die Leiterplatte (3) eine Stärke im Bereich von 0,03 mm bis 0,1 mm aufweist.

13. Katheter nach einem der vorhergehenden Ansprüche, **dadurch gekennzeichnet, dass** der Katheterschaft (10, 110a, 110b) ein Außenschaft (10a) eines Ballonkatheters ist und der Verbinder (2) zur Herstellung einer fluidleitenden Strömungsverbindung mit einem korrespondierenden weiteren Verbinder ausgebildet ist.

14. Verfahren zum Herstellen eines Katheters (1), aufweisend die Schritte:
- Aufdrucken zumindest eines elektrischen Leiters (11, 111a, 111b) auf eine Außenseite (10b) eines Katheterschaftes (10, 110a, 110b) des Katheters (1) mit einer elektrisch leitfähigen Tinte,
- Bereitstellen einer Leiterplatte (3), die zumindest eine Leiterbahn (12) aufweist,
- Anordnen der Leiterplatte (3) auf der Außenseite (10b) des Katheterschaftes (10, 110a, 110b) im Bereich eines proximalen Endabschnitts (10a) des Katheterschaftes (10, 110a, 110b), so dass die Leiterbahn (12) den mindestens einen elektrischen Leiter (11, 111a, 111b) kontaktiert, und
- Umgeben der Leiterplatte (3) und des proximalen Endabschnitts (10a) des Katheterschaftes (10, 110a, 110b) mit einem Material zum Ausbilden eines mit dem proximalen Endabschnitt (10a) des Katheterschaftes (10, 110a, 110b) verbundenen Verbinders (2, 102a, 102b).

15. Verfahren nach Anspruch 14, wobei ein Steckverbinder (4, 104a, 104b) mit der Leiterplatte (3) verbunden wird, so dass die Leiterbahn (12) über den Steckverbinder (4, 104a, 104b) elektrisch kontaktierbar ist, wobei insbesondere der Steckverbinder (4, 104a, 104b) mit der Leiterplatte (3) verbunden wird, bevor die Leiterplatte (3) auf der Außenseite (10b) des proximalen Endabschnitts (10a) des Katheterschaftes (10, 110a, 110b) angeordnet wird.

16. Verfahren nach Anspruch 14 oder 15, wobei zum Umgeben des proximalen Endabschnitts (10a) des Katheterschaftes (10, 110a, 110b) und der Leiterplatte (3) mit dem besagten Material der proximale Endabschnitt (10a) des Katheterschaftes (10, 110a, 110b) und die Leiterplatte (3) in einer Spritzgussform angeordnet werden und in der Spritzgussform mit dem Material umspritzt werden.

## Claims

1. A catheter (1) comprising:
- a catheter shaft (10, 110a, 110b) extending from a proximal end portion (10a) to a distal end portion of the catheter shaft (10, 110a, 110b) and having an outer surface (10b), and
- a connector (2, 120a, 120b) connected to the proximal end portion (10a) of the catheter shaft (10, 110a, 110b),
**characterised in that**
the connector (2, 120a, 120b) comprises a material with which the proximal end portion (10a) of the catheter shaft (10, 110a, 110b) is surrounded, at least one electrical conductor (11, 111a, 111b) being printed on the outer surface (10b) of the catheter shaft (10, 110a, 110b), the at least one electrical conductor (11, 111a, 111b) contacting a conductor track (12) of a printed circuit board (3) which is embedded in the material of the connector (2, 120a, 120b), so that the at least one electrical conductor (11, 111a, 111b) and the at least one conductor track (12) are electrically conductively connected.

2. The catheter according to claim 1, **characterised in that** the at least one electrical conductor (11, 111a, 111b) is formed from a cured electrically conductive ink.

3. The catheter according to claim 1 or 2, **characterised in that** the proximal end portion (10a) of the catheter shaft (10, 110a, 110b) is overmoulded with the material of the connector (2, 120a, 120b).

4. The catheter according to any one of the preceding claims, **characterised in that** the material of the connector (2, 120a, 120b) is a thermoplastic.

5. The catheter according to any one of the preceding claims, **characterised in that** the at least one electrical conductor (11, 111a, 111b) and the conductor track (12) are arranged overlapping.

6. The catheter according to claim 5, **characterised in that** the conductor track (12) is arranged on a surface (3a) of the printed circuit board (3) in a region (12a) of the conductor track (12) in which the conductor track (12) overlaps with the at least one electrical conductor (11, 111a, 111b), the surface (3a) facing the outer surface (10b) of the catheter shaft (10, 110a, 110b).

7. The catheter according to any one of the preceding claims, **characterised in that** the printed circuit board (3) has a first and a second portion (30, 31) , connected thereto, the two portions (30, 31) being arranged at an angle to each other.

8. The catheter according to claim 6 or according to claim 7 if referred back to claim 6, **characterised in that** said region (12a) of the conductor track (12) is situated at an end of the first portion (30).

9. The catheter according to any one of the preceding claims, **characterised in that** the printed circuit board (3) is connected to a plug-in connector (4, 104a, 104b), via which the conductor track (12) may be contacted electrically conductively.

10. The catheter according to claims 7 and 9, **characterised in that** the plug-in connector (4, 104a, 104b) is connected to an end portion of the second portion (31) of the printed circuit board (3).

11. The catheter according to claim 6 or according to any one of claims 7 to 10 if referred back to claim 6, **characterised in that** the conductor track (12) in said region (12a) of the conductor track (12) has a width which is greater than a width of the at least one electrical conductor (11, 111a, 111b).

12. The catheter according to any one of the preceding claims, **characterised in that** the printed circuit board (3) is formed from polyimide and/or **in that** the printed circuit board (3) has a thickness in the range of from 0.03 mm to 0.1 mm.

13. The catheter according to any one of the preceding claims, **characterised in that** the catheter shaft (10, 110a, 110b) is an outer shaft (10a) of a balloon catheter and the connector (2) is designed to establish a fluid-conducting flow connection to a corresponding further connector.

14. A method for producing a catheter (1), comprising the steps of:
- printing at least one electrical conductor (11, 111a, 111b) on an outer surface (10b) of a catheter shaft (10, 110a, 110b) of the catheter (1) using an electrically conductive ink,
- providing a printed circuit board (3) which comprises at least one conductor track (12),
- arranging the printed circuit board (3) on the outer surface (10b) of the catheter shaft (10, 110a, 110b) in the region of a proximal end portion (10a) of the catheter shaft (10, 110a, 110b) so that the conductor track (12) contacts the at least one electrical conductor (11, 111a, 111b), and
- surrounding the printed circuit board (3) and the proximal end portion (10a) of the catheter shaft (10, 110a, 110b) with a material for forming a connector (2, 102a, 102b) connected to the proximal end portion (10a) of the catheter shaft (10, 110a, 110b).

15. The method according to claim 14, wherein a plug-in connector (4, 104a, 104b) is connected to the printed circuit board (3) so that the conductor track (12) may be electrically contacted via the plug-in connector (4, 104a, 104b), wherein in particular the plug-in connector (4, 104a, 104b) is connected to the printed circuit board (3) before the printed circuit board (3) is arranged on the outer surface (10b) of the proximal end portion (10a) of the catheter shaft (10, 110a, 110b).

16. The method according to claim 14 or 15, wherein, in order to surround the proximal end portion (10a) of the catheter shaft (10, 110a, 110b) and the printed circuit board (3) with said material, the proximal end portion (10a) of the catheter shaft (10, 110a, 110b) and the printed circuit board (3) are arranged in an injection mould and are overmoulded with the material in the injection mould.

## Revendications

1. Cathéter (1) muni
- d'une tige de cathéter (10, 110a, 110b) qui s'étend à partir d'un segment d'extrémité proximal (10a) jusqu'à un segment d'extrémité distal de la tige de cathéter (10, 110a, 110b) et présente une face extérieure (10b), et
- d'une jonction (2, 120a, 120b) reliée avec le segment d'extrémité proximal (10a) de la tige de cathéter (10, 110a, 110b),
**caractérisé en ce que**
la jonction (2, 120a, 120b) présente un matériau dont est entouré le segment d'extrémité proximal (10a) de la tige de cathéter (10, 110a, 110b), au moins un conducteur électrique (11, 111a, 111b) étant imprimé sur la face extérieure (10b) de la tige de cathéter (10, 110a, 110b), l'au moins un conducteur électrique (11, 111a, 111b) étant en contact avec une piste conductrice (12) d'une plaque conductrice (3) qui est incorporée dans le matériau de la jonction (2, 120a, 120b), de sorte que l'au moins un conducteur électrique (11, 111a, 111b) et l'au moins une piste conductrice (12) sont reliés électriquement de manière conductrice.

2. Cathéter selon la revendication 1, **caractérisé en ce que** l'au moins un conducteur électrique (11, 111a, 111b) est formé à base d'une encre conductrice durcie électriquement.

3. Cathéter selon la revendication 1 ou la revendication 2, **caractérisé en ce que** le segment d'extrémité proximal (10a) de la tige de cathéter (10, 110a, 110b) a été pulvérisé tout autour avec le matériau de la jonction (2, 120a, 120b).

4. Cathéter selon l'une des revendications précédentes, **caractérisé en ce que** le matériau de la jonction (2, 120a, 120b) est à base d'une matière thermoplastique.

5. Cathéter selon l'une des revendications précédentes, **caractérisé en ce que** l'au moins un conducteur électrique (11, 111a, 111b) et la piste conductrice (12) sont disposés de manière superposée.

6. Cathéter selon la revendication 5, **caractérisé en ce que** la piste conductrice (12), dans une plage (12a) de la piste conductrice (12), dans laquelle la piste conductrice (12) se superpose avec l'au moins un conducteur électrique (11, 111a, 111b), est disposée sur une surface (3a) de la plaque conductrice (3), la surface (3a) étant orientée vers la face extérieure (10b) de la tige de cathéter (10, 110a, 110b).

7. Cathéter selon l'une des revendications précédentes, **caractérisé en ce que** la plaque conductrice (3) présente un premier segment et un deuxième segment (30, 31) relié à celui-ci, les deux segments (30, 31) étant disposés en faisant un angle l'un par rapport à l'autre.

8. Cathéter selon la revendication 6 ou selon la revendication 7, dans la mesure où elle est reliée avec la revendication 7, **caractérisé en ce que** ladite plage (12a) de la piste conductrice (12) est disposée à une extrémité du premier segment (30).

9. Cathéter selon l'une des revendications précédentes, **caractérisé en ce que** la plaque conductrice (3) est reliée avec un connecteur (4, 104a, 104b) par lequel la piste conductrice (12) peut être mise en contact de manière électriquement conductrice.

10. Cathéter selon les revendications 7 et 9, **caractérisé en ce que** le connecteur (4, 104a, 104) est relié avec un segment d'extrémité du deuxième segment (31) de la plaque conductrice (3).

11. Cathéter selon la revendication 6 ou selon l'une des revendications 7 à 10, dans la mesure où elles sont reliées à la revendication 6, **caractérisé en ce que** la piste conductrice (12) présente, dans ladite plage (12a) de la piste conductrice (12), une largeur qui est supérieure à une largeur de l'au moins un conducteur électrique (11, 111a, 111b).

12. Cathéter selon l'une des revendications précédentes, **caractérisé en ce que** la plaque conductrice (3) est constituée de polyimide et/ou la plaque conductrice (3) présente une épaisseur dans la plage de 0,03 mm à 0,1 mm.

13. Cathéter selon l'une des revendications précédentes, **caractérisé en ce que** la tige de cathéter (10, 110a, 110b) est une tige extérieure (10a) d'un cathéter de ballonnet et la jonction (2) est conçue pour l'établissement d'une liaison d'écoulement conductrice de fluide avec une autre jonction correspondante.

14. Procédé de fabrication d'un cathéter (1) présentant les étapes :
- d'impression d'au moins un conducteur électrique (11, 111a, 111b) sur une face extérieure (10b) d'une tige de cathéter (10, 110a, 110b) du cathéter (1) avec une encre électriquement conductrice,
- de mise en place d'une plaque conductrice (3) qui présente au moins une piste conductrice (12),
- de disposition de la plaque conductrice (3) sur la face extérieure (10b) de la tige de cathéter (10, 110a, 110b) dans la zone d'un segment d'extrémité proximal (10a) de la tige de cathéter (10, 110a, 110b) de sorte que la piste conductrice (12) entre en contact avec l'au moins un conducteur électrique (11, 111a, 111b), et
- l'entourage de la plaque conductrice (3) et du segment d'extrémité proximal (10a) de la tige de cathéter (10, 110a, 110b) avec un matériau pour la formation d'une jonction (2, 102a, 102b) reliée avec le segment d'extrémité proximal (10a) de la tige de cathéter (10, 110a, 110b).

15. Procédé selon la revendication 14, dans lequel un connecteur (4, 104a, 104b) est relié avec la plaque conductrice (3) de sorte que la piste conductrice (12) puisse être mise en contact via le connecteur (4, 104a, 104b), dans lequel, en particulier, le connecteur (4, 104a, 104b) est relié avec la plaque conductrice (3) avant que la plaque conductrice (3) ne soit disposée sur la face extérieure (10b) du segment d'extrémité proximal (10a) de la tige de cathéter (10, 110a, 110b).

16. Procédé selon la revendication 14 ou la revendication 15, dans lequel, pour l'entourage du segment d'extrémité proximal (10a) de la tige de cathéter (10, 110a, 110b) et de la plaque conductrice (3) avec ledit matériau, le segment d'extrémité proximal (10a) de la tige de cathéter (10, 110a, 110b) et la plaque conductrice (3) sont disposés dans un moule d'injection et sont pulvérisés tout autour dans le moule d'injection avec le matériau.
